# EUROPÄISCHE PATENTSCHRIFT

(11) **EP 2 550 065 B1**
(45) Veröffentlichungstag und Bekanntmachung des Hinweises auf die Patenterteilung: **13.09.2017**
(21) Anmeldenummer: 11700670.0
(22) Anmeldetag: 21.01.2011
(51) Int. Cl.: A61K 8/81, A61Q 5/10

(54) **VERDICKENDE OXIDATIONSZUBEREITUNGEN**
THICKENED OXIDATION PREPARATIONS
PRÉPARATIONS D'OXYDATION À EFFET ÉPAISSISSANT

(30) Priorität: 25.03.2010 DE 102010003263
(43) Veröffentlichungstag der Anmeldung: 30.01.2013
(73) Patentinhaber: Henkel AG & Co. KGaA, 40589 Düsseldorf (DE)
(72) Erfinder: GOUTSIS, Konstantin, 41812 Erkelenz (DE); JANßEN, Frank, 41470 Neuss (DE); KRIPPAHL, Marc, 40789 Monheim (DE)
(86) Internationale Anmeldenummer: PCT/EP2011/050802
(87) Internationale Veröffentlichungsnummer: WO 2011/116994

(56) Entgegenhaltungen:
- EP-A2- 1 430 874
- WO-A1-2005/067874
- US-A1- 2008 083 420
- US-A1- 2009 241 272

## Beschreibung

Gegenstand der vorliegenden Anmeldung ist eine kosmetisches Mittel zur oxidativen Farbveränderung von keratinischen Fasern, insbesondere menschliche Haaren, welche unmittelbar vor der Anwendung aus zwei Zubereitungen zusammengemischt wird, wobei eine der Zubereitungen Wasserstoffperoxid und mindestens eine vernetzte Polyacrylsäure enthält.

Zur Bereitstellung farbverändernder kosmetischer Mittel, insbesondere für die Haut oder keratinhaltige Fasern wie beispielsweise menschliche Haare, kennt der Fachmann je nach Anforderungen an die Färbung diverse Färbesysteme. Für permanente, intensive Färbungen mit entsprechenden Echtheitseigenschaften werden sogenannte Oxidationsfärbemittel verwendet. Solche Färbemittel enthalten üblicherweise Oxidationsfarbstoffvorprodukte, sogenannte Entwicklerkomponenten und Kupplerkomponenten, die unter dem Einfluss von Oxidationsmitteln oder von Luftsauerstoff untereinander die eigentlichen Farbstoffe ausbilden. Die Oxidationsfärbemittel zeichnen sich durch hervorragende, lang anhaltende Färbeergebnisse aus. Neben der Färbung ist das Aufhellen der eigenen Haarfarbe bzw. das Blondieren der ganz spezielle Wunsch vieler Verbraucher. Dazu werden die die Faser färbenden natürlichen oder künstlichen Farbstoffe meist oxidativ unter Einsatz von entsprechenden Oxidationsmitteln, wie beispielsweise Wasserstoffperoxid, entfärbt. Für temporäre Färbungen werden üblicherweise Färbe- oder Tönungsmittel verwendet, die als färbende Komponente sogenannte direktziehende Farbstoffe ("Direktzieher") enthalten. Diese lassen sich ebenfalls zusammen mit Oxidationsmitteln in aufhellenden Färbemitteln einsetzen.

Um eine optimale Färbeleistung zu entfalten, benötigen oxidative Färbemittel in der Regel einen alkalischen pH-Wert zur Ausfärbung, insbesondere zwischen pH 9,0 und pH 11,5. Aus Stabilitätsgründen werden oxidative Färbemittel üblicherweise erst unmittelbar vor der Anwendung durch Vermischen einer Farbveränderungszubereitung und einer Oxidationsmittelzubereitung hergestellt. Die Farbveränderungszubereitung besitzt zur Stabilisierung von Oxidationsfarbstoffvorprodukten zumeist einen alkalischen pH-Wert und die Oxidationsmittelzubereitung besitzt zur Stabilisierung des Oxidationsmittels einen sauren pH-Wert, während die Anwendungsmischung einen alkalischen pH-Wert besitzen sollte, um eine gute Penetration der Farbstoffvorprodukte und der Oxidationsmittel in die keratinische Faser zu ermöglichen.

Darüber hinaus beträgt die Anwendungsdauer für ansprechende Färbeergebnisse üblicherweise zwischen 10 und 60 min. Das anwendungsbereite Farbveränderungsmittel sollte daher so formuliert und konfektioniert sein, dass das Färbemittel sich einerseits gut auf den zu färbenden keratinischen Fasern verteilen lässt, andererseits jedoch in den zu färbenden Fasern während der Anwendungszeit verbleibt. Dazu ist es vorteilhaft, wenn das Färbemittel über eine bestimmte Viskosität verfügt, die zwar das Auftragen des Mittels ermöglicht, jedoch das Mittel auch am Ort der Anwendung verbleiben lässt. Diese Viskosität kann durch polymere Verdickungsmittel im anwendungsbereiten Farbveränderungsmittel eingestellt werden, wobei dieses Verdickungsmittel sowohl in der Farbveränderungszubereitung oder der Oxidationsmittelzubereitung enthalten sein kann.

Um eine gute Vermischung von Farbveränderungszubereitung und Oxidationsmittelzubereitung zu ermöglichen, ist es vorteilhaft, wenn die Farbveränderungszubereitung und Oxidationsmittelzubereitung über eine gute Fließfähigkeit verfügen und sich die erhöhte Viskosität der Anwendungsmischung erst nach Vermischen der beiden Komponenten einstellt. Gängige polymere Verdickungsmitteln in kosmetischen Zubereitungen sind Derivate von Cellulosen, wie Hydroxyethylcellulose oder Xanthan.

Aufgabe der vorliegenden Erfindung ist es daher, ein farbveränderndes Mittel zur Verfügung zu stellen, durch welches die oben genannten Nachteile gebräuchlicher farbverändernden Mittel herabgesenkt werden. Insbesondere ist es die Aufgabe der vorliegenden Erfindung, ein oxidatives Farbveränderungsmittel für keratinische Fasern bereitzustellen, welches sich durch eine sehr gute Mischbarkeit der beiden Komponenten Oxidationsmittelzubereitung und Farbveränderungszubereitung auszeichnet. Die daraus resultierende Anwendungsmischung soll eine ausreichende Viskosität aufweisen, so dass das Mittel sich einerseits gut auftragen lässt, andererseits während der Anwendung am Wirkort verbleibt und nicht aus den Fasern ausfließt.

Eine Möglichkeit zur Erzielung einer ausreichenden Verdickung ist der Einsatz von polymeren Verdickungsmitteln, deren verdickende Eigenschaften sich mit dem pH-Wert ändern. Diese Eigenschaft lässt sich besonders vorteilhaft nutzen, wenn der polymere Verdicker in der sauren Oxidationsmittelzubereitung enthalten ist, da dieses Mittel beim Vermischen zur Anwendungszubereitung einen großen pH-Wert-Wechsel erfährt. Daher ist ein anionischer polymerer Verdicker bevorzugt, der bei einem alkalischen pH-Wert zu einer deutlichen Viskositätserhöhung führt. Als solche anionischen, polymeren Verdickungsmittel eignen sich besonders Homo- oder Copolymere von Acrylsäure oder Methacrylsäure.

EP 1430874A2 offenbart oxidative Haarfärbemittel, deren Oxidationsmittelzubereitungen ein Copolymer mit der INCI-Bezeichnung "Acrylates/Steareth-20 Methacrylate Copolymer" oder ein Copolymer mit der INCI-Bezeichnung "Acrylates Copolymer" enthalten. US 2008/083420A1 offenbart ein oxidatives Haarfärbemittel ohne direktziehende Farbstoffe, dessen Oxidationsmittelzubereitung Carbopol 956 enthält. US 2009/0241272A1 offenbart oxidative Haarfärbemittel, deren Oxidationsmittelzubereitungen ein nicht näher definiertes Carbomer enthalten. WO 2005/067874A1 offenbart Oxidationsmittelzubereitungen für oxidative Haarfärbe- und -blondiermittel, die diverse Copolymere enthalten.

In überraschender Weise wurde jedoch gefunden, dass sich bestimmte vernetzte Polyacrylsäuren in der Oxidationsmittelzubereitung ganz besonders vorteilhaft zur Einstellung der Viskosität einsetzen lassen. Die Anmelderin hat in ihren Untersuchungen unerwartet gefunden, dass sich dabei insbesondere auch die Menge des einzusetzenden Verdickungsmittels reduzieren lässt, ohne Einbußen in der Verdickung hinnehmen zu müssen. Damit besitzt die Oxidationsmittelzubereitung eine gute Fließfähigkeit, die eine gute Durchmischung mit der Farbveränderungszubereitung, beispielsweise durch Schütteln, ermöglicht. Schließlich hat die Verringerung der Einsatzmenge an Verdickungsmitteln neben der Rohstoffersparnis auch Vorteile bei der Herstellung der Mittel, da durch geringere Verdickungsmittelmengen die Leitungen und Ventile in der Herstellanlage weniger zu Verstopfungen neigen und die Reinigung der Anlagen erleichtert wird.

Ein erster Gegenstand der vorliegenden Erfindung ist daher ein kosmetisches Mittel für die Farbveränderung keratinischer Fasern,
welches unmittelbar vor der Anwendung durch Vermischen einer Zubereitung (M1), enthaltend mindestens einen direktziehenden Farbstoff als farbverändernde Komponente, und einer Oxidationsmittelzubereitung (M2), enthaltend in einem wässrigen, kosmetischen Träger als chemisches Oxidationsmittel mindestens Wasserstoffperoxid, hergestellt wird und welches eine Viskosität von 5 bis 100 Pa·s, bevorzugt von 10 bis 30 Pa·s, besitzt, gemessen bei 22°C, und welches dadurch gekennzeichnet ist, dass die Oxidationsmittelzubereitung zusätzlich mindestens ein anionisches, polymeres Verdickungsmittel, ausgewählt aus vernetzten Polyacrylsäuren, die ein Molekulargewicht MW von mindestens 500000 g/mol besitzen, enthält, wobei die Polyacrylsäuren Polymerisate aus Acrylsäure selbst darstellen, und weiterhin dadurch gekennzeichnet ist, dass die Zubereitung (M1) zusätzlich mindestens ein Alkalisierungsmittel, ausgewählt aus Ammoniak, Natriumhydroxid, Natriumcarbonat, Kaliumhydroxid, Kaliumcarbonat, Natriumhydrogencarbonat, Ammoniumcarbonat, Ammoniumhydrogencarbonat, Arginin, Histidin, Monoethanolamin und/oder 2-Amino-2-methylpropanol, enthält.

Unter keratinhaltigen bzw. keratinischen Fasern werden erfindungsgemäß Pelze, Wolle, Federn und insbesondere menschliche Haare verstanden. Obwohl die erfindungsgemäßen Mittel in erster Linie zum Färben von keratinhaltigen Fasern geeignet sind, steht prinzipiell einer Verwendung auch auf anderen Gebieten nichts entgegen.

Die erfindungsgemäßen Zubereitungen enthalten die Wirkstoffe in einem kosmetischen Träger. Dieser kosmetische Träger ist im Sinne der Erfindung wässrig, alkoholisch oder wässrig-alkoholisch. Unter wässrig-alkoholischen Trägern sind im Sinne der vorliegenden Erfindung wasserhaltige Zusammensetzungen, enthaltend 3 bis 70 Gew.-% eines C₁-C₄-Alkohols, bezogen auf das Gesamtgewicht der Anwendungsmischung, insbesondere Ethanol bzw. Isopropanol, zu verstehen. Die erfindungsgemäßen Mittel können zusätzlich weitere organische Lösungsmittel, wie beispielsweise 4-Methoxybutanol, Ethyldiglykol, 1,2-Propylenglykol, n-Propanol, n-Butanol, n-Butylenglykol, Glycerin, Diethylenglykolmonoethylether, und Diethylenglykolmono-n-butylether, enthalten. Bevorzugt sind dabei alle wasserlöslichen organischen Lösungsmittel. Ein wässriger Träger enthält im Sinne der Erfindung mindestens 30 Gew.-%, insbesondere mindestens 50 Gew.-% Wasser, bezogen auf das Gesamtgewicht der Zubereitung. Zum Zwecke der Haarfärbung sind solche Träger beispielsweise Cremes, Emulsionen, Gele oder auch tensidhaltige schäumende Lösungen, wie beispielsweise Shampoos, Schaumaerosole oder andere Zubereitungen, die für die Anwendung auf dem Haar geeignet sind. Bevorzugte Träger stellen dabei Emulsionen und Gele dar, wobei Emulsionen besonders bevorzugt sind.

Die anwendungsbereiten Färbemittel besitzen erfindungsgemäß eine hinreichende Viskosität, die es ermöglicht, dass das Mittel einerseits sich gut auf die zu färbenden Fasern auftragen lässt und am Wirkort verteilen lässt, andererseits aber in zu färbenden Region auf der keratinischen Faser verbleibt und nicht ausfließt. Dazu besitzen die erfindungsgemäßen Mittel eine Viskosität von 5 bis 100 Pa·s, bevorzugt von 10 bis 30 Pa·s und besonders bevorzugt von 12 bis 25 Pa·s. Die hier angeführten, erfindungsgemäßen und bevorzugten Viskositäten beziehen sich dabei jeweils auf Viskositäten, welche mit einem Rotationsviskosimeter (Brookfield, 22°C, Spindel #5, 4 rpm) bestimmt wurden.

Das kosmetische Mittel wird vor der Anwendung durch Vermischen einer Farbveränderungszubereitung (M1) mit einer Oxidationsmittelzubereitung (M2) hergestellt.

Erfindungsgemäß enthält die Farbveränderungszubereitung (M1) dabei mindestens einen direktziehenden Farbstoff als farbverändernde Komponente. Als farbverändernde Komponente in Mittel (M1) können Aufhellmittel als zusätzliche Bleichkraftverstärker, die die Wirkung des Oxidationsmittels verstärken, sowie farbgebende Komponenten eingesetzt werden.

In einer Ausführungsform enthält das erfindungsgemäße Mittel (M1) daher einen zusätzlichen Bleichkraftverstärker. Als zusätzliche Bleichkraftverstärker können im Rahmen dieser Erfindung Peroxoverbindungen, weiterhin Verbindungen, die unter Perhydrolysebedingungen aliphatische Peroxocarbonsäuren und/oder substituierte Perbenzoesäure ergeben, Kohlensäurederivate, Alkylcarbonate, -carbamate, Silylcarbonate und -carbamate eingesetzt werden.
Vorzugsweise ist der Bleichkraftverstärker ausgewählt aus Ammoniumperoxodisulfat, Alkalimetallperoxodisulfaten, Ammoniumperoxomonosulfat, Alkalimetallhydrogenperoxomonosulfaten, Alkalimetallperoxodiphosphaten und Erdalkalimetallperoxiden. Besonders bevorzugte Bleichkraftverstärker sind Ammoniumperoxodisulfat, Kaliumperoxodisulfat, Natriumperoxodisulfat, Kaliumhydrogenperoxomonosulfat, Kaliumperoxodiphosphat, Magnesiumperoxid und Bariumperoxid. Erfindungsgemäß besonders bevorzugt sind Mittel, die als Bleichkraftverstärker mindestens ein anorganisches Salz, ausgewählt aus Peroxomonosulfaten und/oder Peroxodisulfaten, enthalten. Weiter hin hat es sich bei den Arbeiten zur vorliegenden Erfindung als besonders bevorzugt erwiesen, wenn die erfindungsgemäßen Mittel mindestens zwei verschiedene Peroxodisulfate enthalten. Bevorzugte Peroxodisulfatsalze sind dabei Kombinationen aus Ammoniumperoxodisulfat und Kaliumperoxodisulfat und/oder Natriumperoxodisulfat. Die Peroxoverbindungen sind in einer Menge von 0,1 bis 25 Gew.-%, insbesondere in einer Menge von 0,5 bis 15 Gew.-%, bezogen auf das Gesamtgewicht des anwendungsbereiten Mittels, enthalten.
Der Einsatz von Persulfatsalzen bzw. Peroxodisulfatsalzen erfolgt in der Regel wasserfrei und in Form eines gegebenenfalls entstaubten Pulvers, Paste oder eines in Form gepressten Formkörpers. Die wasserfreien Mittel (M1) können anstelle und/oder zusätzlich zu den festen Peroxoverbindungen einen weiteren Bleichkraftverstärker enthalten.

Obwohl prinzipiell keine Einschränkungen hinsichtlich der Formulierung des Mittel (M1) bestehen, hat es sich erfindungsgemäß als bevorzugt erwiesen, dass das Mittel (M1) wasserfrei formuliert ist, wenn das Mittel (M1) als farbverändernde Komponente einen zusätzlichen Bleichkraftverstärker enthält. Wasserfrei im Sinne der vorliegenden Erfindung bedeutet einen Wassergehalt bezogen auf die Mittel (M1) von weniger als 5 Gew.-%, insbesondere von weniger als 2 Gew.-%. Mittel (M1), die weniger als 0,1 Gew.-% Wasser, enthalten, können erfindungsgemäß besonders bevorzugt sein. Mittel (M1) ist vorzugsweise als Pulver oder als wasserfreie Paste formuliert.

In einer weiteren, bevorzugten Ausführungsform kann das Mittel (M1) als Bleichkraftverstärker mindestens ein kationisches Pyridinium-Derivat enthalten. Bevorzugte Verbindungen sind 4-AcylPyridinium-Derivate und 2-Acylpyridinium-Derivate. Insbesondere bevorzugt sind dabei 2-Acetyl-1-methylpyridinium-p-toluolsulfonat und 4-Acetyl-1-methylpyridinium-p-toluolsulfonat. Weiterhin bevorzugte kationische Pyridinium-Derivate sind dabei kationische 3,4-Dihydroisochinolinium-Derivat. Insbesondere bevorzugt ist N-Methyl-3,4-dihydroisochinolinium-p-toluolsulfonat.

Die neben oder anstelle von Peroxoverbindungen eingesetzten Bleichkraftverstärker sind in den erfindungsgemäßen, kosmetischen Mitteln bevorzugt in Mengen von 0,05 bis 10 Gew.-%, insbesondere in Mengen von 0,2 bis 5 Gew.-%, jeweils bezogen auf das Gesamtgewicht des anwendungsbereiten Mittels, enthalten.

Zur weiteren Steigerung der Aufhellleistung kann der erfindungsgemäßen Zubereitung (M1) zusätzlich als Bleichverstärker mindestens eine gegebenenfalls hydratisierte SiO₂-Verbindung zugesetzt werden. Obwohl bereits geringe Mengen der gegebenenfalls hydratisierten SiO₂-Verbindunge die Aufhellleistung erhöhen, kann es erfindungsgemäß bevorzugt sein, die gegebenenfalls hydratisierten SiO₂-Verbindungen in Mengen von 0,05 Gew.-% bis 15 Gew.-%, besonders bevorzugt in Mengen von 0,15 Gew.-% bis 10 Gew.-% und ganz besonders bevorzugt in Mengen von 0,2 Gew.-% bis 5 Gew.-%, jeweils bezogen auf die erfindungsgemäße wasserfreie Zusammensetzung, einzusetzen. Die Mengenangaben geben dabei jeweils den Gehalt der SiO₂-Verbindun (ohne deren Wasseranteil) in den Mitteln wieder. Bevorzugte gegebenenfalls hydratisierten SiO₂-Verbindunge sind Kieselsäuren, deren Oligomeren und Polymeren sowie deren Salze. Die gegebenenfalls hydratisierten SiO₂-Verbindunge können in verschiedenen Formen vorliegen. Erfindungsgemäß bevorzugt werden die SiO₂-Verbindunge in Form von Kieselgelen (Silicagel) oder besonders bevorzugt als Wasserglas eingesetzt. Erfindungsgemäß bevorzugt sind Wassergläser, die aus einem Silikat der Formel (SiO₂)ₙ(Na₂O)ₘ(K₂O)ₚ gebildet werden, wobei n steht für eine positive rationale Zahl und m und p stehen unabhängig voneinander für eine positive rationale Zahl oder für 0, mit den Maßgaben, dass mindestens einer der Parameter m oder p von 0 verschieden ist und das Verhältnis zwischen n und der Summe aus m und p zwischen 1:4 und 4:1 liegt. Insbesondere Metasilicate, die sich gemäß vorstehender Formel durch das Verhältnis zwischen n und der Summe aus m und p von ≤ 1 auszeichnen und sich als kettenförmige polymere Strukturen des Anions [SiO₃]²⁻ auffassen lassen, können bevorzugt eingesetzt werden. Natriummetasilicat der Formel [NaSiO₃]ₓ ist dabei besonders bevorzugt.

Erfindungsgemäß bevorzugte Mittel zum Farbveränderung keratinischer Fasern sind dadurch gekennzeichnet, dass sie mindestens ein Oxidationsfarbstoffvorprodukt enthalten. Die erfindungsgemäß bevorzugten Aufhellmittel enthalten als Oxidationsfarbstoffvorprodukt mindestens ein Oxidationsfarbstoffvorprodukt vom Entwicklertyp (Entwicklerkomponente), bevorzugt in Kombination mit mindestens einer Oxidationsfarbstoffvorprodukt vom Kupplertyp (Kupplerkomponente).

Bevorzugte Oxidationsfarbstoffvorprodukte vom Entwicklertyp sind p-Phenylendiaminderivate. Bevorzugte p-Phenylendiamine werden ausgewählt aus einer oder mehrerer Verbindungen der Gruppe, die gebildet wird, aus p-Phenylendiamin, p-Toluylendiamin, 2-Chlor-p-phenylendiamin, 2,3-Dimethyl-p-phenylendiamin, 2,6-Dimethyl-p-phenylendiamin, 2,6-Diethyl-p-phenylendiamin, 2,5-Dimethyl-p-phenylendiamin, N,N-Dimethyl-p-phenylendiamin, N,N-Diethyl-p-phenylendiamin, N,N-Dipropyl-p-phenylendiamin, 4-Amino-3-methyl-(N,N-diethyl)anilin, N,N-Bis-(2-hydroxyethyl)-p-phenylendiamin, 4-N,N-Bis-(2-hydroxyethyl)amino-2-methylanilin, 4-N,N-Bis-(2-hydroxyethyl)-amino-2-chloranilin, 2-(2-Hydroxyethyl)-p-phenylendiamin, 2-(1,2-Dihydroxyethyl)-p-phenylendiamin, 2-Fluor-p-phenylendiamin, 2-Isopropyl-p-phenylendiamin, N-(2-Hydroxypropyl)-p-phenylendiamin, 2-Hydroxymethyl-p-phenylendiamin, N,N-Dimethyl-3-methyl-p-phenylendiamin, N-Ethyl-N-2-hydroxyethyl-p-phenylendiamin, N-(2,3-Dihydroxypropyl)-p-phenylendiamin, N-(4'-Aminophenyl)-p-phenylendiamin, N-Phenyl-p-phenylendiamin, 2-(2-Hydroxyethyloxy)-p-phenylendiamin, 2-Methoxymethyl-p-phenylendiamin, 2-(2-Acetylaminoethyloxy)-p-phenylendiamin, N-(2-Methoxyethyl)-p-phenylendiamin, N-(4-Amino-3-methylphenyl)-N-[3-(1H-imidazol-1-yl)propyl]amin, 5,8-Diamino-benzo-1,4-dioxan sowie ihren physiologisch verträglichen Salzen. Erfindungsgemäß besonders bevorzugte p-Phenylendiaminderivate sind ausgewählt aus mindestens einer Verbindung der Gruppe p-Phenylendiamin, p-Toluylendiamin, 2-(2-Hydroxyethyl)-p-phenylendiamin, 2-(1,2-Dihydroxyethyl)-p-phenylendiamin, N,N-Bis-(2-hydroxyethyl)-p-phenylendiamin, N-(4-Amino-3-methylphenyl)-N-[3-(1H-imidazol-1-yl)propyl]amin, 2-Methoxymethyl-p-phenylendiamin sowie deren physiologisch verträglichen Salzen.

Es kann erfindungsgemäß weiterhin bevorzugt sein, als Entwicklerkomponente Verbindungen einzusetzen, die mindestens zwei aromatische Kerne enthalten, die mit Amino- und/oder Hydroxylgruppen substituiert sind. Bevorzugte zweikernige Entwicklerkomponenten werden insbesondere aus mindestens einer der folgenden Verbindungen ausgewählt: N,N'-Bis-(2-hydroxyethyl)-N,N'-bis-(4'-aminophenyl)-1,3-diaminopropan-2-ol, N,N'-Bis-(2-hydroxyethyl)-N,N'-bis-(4'-aminophenyl)-ethylendiamin, N,N'-Bis-(4'-aminophenyl)tetramethylendiamin, N,N'-Bis-(2-hydroxyethyl)-N,N'-bis-(4'-aminophenyl)-tetramethylendiamin, N,N'-Bis-(4-(methylamino)phenyl)tetramethylendiamin, N,N'-Diethyl-N,N'-bis-(4'-amino-3'-methylphenyl)ethylendiamin, Bis-(2-hydroxy-5-aminophenyl)methan, N,N'-Bis-(4'-aminophenyl)-1,4-diazacycloheptan, N,N'-Bis-(2-hydroxy-5-aminobenzyl)piperazin, N-(4'-Aminophenyl)-p-phenylendiamin und 1,10-Bis-(2',5'-diaminophenyl)-1,4,7,10-tetraoxadecan sowie ihre physiologisch verträglichen Salze. Besonders bevorzugte zweikernige Entwicklerkomponenten werden ausgewählt unter N,N'-Bis-(2-hydroxyethyl)-N,N'-bis-(4-aminophenyl)-1,3-diamino-propan-2-ol, Bis-(2-hydroxy-5-aminophenyl)methan, 1,3-Bis-(2,5-diaminophenoxy)-propan-2-ol, N,N'-Bis-(4-aminophenyl)-1,4-diazacycloheptan, 1,10-Bis-(2,5-diaminophenyl)-1,4,7,10-tetraoxadecan oder deren physiologisch verträglichen Salzen.

Weiterhin kann es erfindungsgemäß bevorzugt sein, als Entwicklerkomponente ein p-Aminophenolderivat oder eines seiner physiologisch verträglichen Salze einzusetzen. Bevorzugte p-Aminophenole sind insbesondere p-Aminophenol, N-Methyl-p-aminophenol, 4-Amino-3-methyl-phenol, 4-Amino-3-fluorphenol, 2-Hydroxymethylamino-4-aminophenol, 4-Amino-3-hydroxymethylphenol, 4-Amino-2-(2-hydroxyethoxy)-phenol, 4-Amino-2-methylphenol, 4-Amino-2-hydroxymethylphenol, 4-Amino-2-methoxymethyl-phenol, 4-Amino-2-aminomethylphenol, 4-Amino-2-(2-hydroxyethyl-aminomethyl)-phenol, 4-Amino-2-(1,2-dihydroxyethyl)-phenol, 4-Amino-2-fluorphenol, 4-Amino-2-chlorphenol, 4-Amino-2,6-dichlorphenol, 4-Amino-2-(diethylaminomethyl)phenol sowie ihre physiologisch verträglichen Salze. Besonders bevorzugte Verbindungen sind p-Aminophenol, 4-Amino-3-methylphenol, 4-Amino-2-aminomethylphenol, 4-Amino-2-(1,2-dihydroxyethyl)phenol und 4-Amino-2-(diethylaminomethyl)phenol.

Ferner kann die Entwicklerkomponente ausgewählt sein aus o-Aminophenol und seinen Derivaten, wie beispielsweise 2-Amino-4-methylphenol, 2-Amino-5-methylphenol oder 2-Amino-4-chlorphenol.

Weiterhin kann die Entwicklerkomponente ausgewählt sein aus heterocyclischen Entwicklerkomponenten, wie beispielsweise aus Pyrimidinderivaten, Pyrazolderivaten, Pyrazolopyrimidin-und Pyrazolopyrazol-Derivaten bzw. ihren physiologisch verträglichen Salzen. Bevorzugte PyrimidinDerivate sind insbesondere die Verbindungen 2,4,5,6-Tetraaminopyrimidin, 4-Hydroxy-2,5,6-triaminopyrimidin, 2-Hydroxy-4,5,6-triaminopyrimidin, 2-Dimethylamino-4,5,6-triaminopyrimidin, 2,4-Dihydroxy-5,6-diaminopyrimidin und 2,5,6-Triaminopyrimidin. Bevorzugte Pyrazol-Derivate sind insbesondere die Verbindungen, die ausgewählt werden unter 4,5-Diamino-1-methylpyrazol, 4,5-Diamino-1-(2-hydroxyethyl)pyrazol, 3,4-Diaminopyrazol, 4,5-Diamino-1-(4'-chlorbenzyl)pyrazol, 4,5-Diamino-1,3-dimethylpyrazol, 4,5-Diamino-3-methyl-1-phenylpyrazol, 4,5-Diamino-1-methyl-3-phenylpyrazol, 4-Amino-1,3-dimethyl-5-hydrazinopyrazol, 1-Benzyl-4,5-diamino-3-methylpyrazol, 4,5-Diamino-3-t-butyl-1-methylpyrazol, 4,5-Diamino-1-t-butyl-3-methylpyrazol, 4,5-Diamino-1-(2-hydroxyethyl)-3-methylpyrazol, 4,5-Diamino-1-ethyl-3-methylpyrazol, 4,5-Diamino-1-ethyl-3-(4-methoxyphenyl)pyrazol, 4,5-Diamino-1-ethyl-3-hydroxymethylpyrazol, 4,5-Diamino-3-hydroxymethyl-1-methylpyrazol, 4,5-Diamino-3-hydroxymethyl-1-isopropylpyrazol, 4,5-Diamino-3-methyl-1-isopropylpyrazol, 4-Amino-5-(2-aminoethyl)amino-1,3-dimethylpyrazol sowie deren physiologisch verträglichen Salze, insbesondere jedoch 4,5-Diamino-1-(2-hydroxyethyl)pyrazol. Bevorzugte Pyrazolopyrimidine sind die Verbindungen, die ausgewählt werden unter Pyrazolo[1,5-a]pyrimidin-3,7-diamin, 2,5-Dimethyl-pyrazolo[1,5-a]pyrimidin-3,7-diamin, Pyrazolo[1,5-a]pyrimidin-3,5-diamin, 2,7-Dimethyl-pyrazolo[1,5-a]pyrimidin-3,5-diamin, 3-Aminopyrazolo[1,5-a]pyrimidin-7-ol, 3-Aminopyrazolo[1,5-a]pyrimidin-5-ol, 2-(3-Aminopyrazolo[1,5-a]pyrimidin-7-ylamino)ethanol, 2-(7-Aminopyrazolo[1,5-a]pyrimidin-3-ylamino)ethanol, 2-((3-Aminopyrazolo[1,5-a]pyrimidin-7-yl)-(2-hydroxyethyl)-amino]ethanol, 2-[(7-Aminopyrazolo[1,5-a]pyrimidin-3-yl)-(2-hydroxyethyl)amino]ethanol, 5,6-Dimethylpyrazolo[1,5-a]pyrimidin-3,7-diamin, 2,6-Dimethylpyrazolo[1,5-a]pyrimidin-3,7-diamin, 3-Amino-7-dimethylamino-2,5-dimethylpyrazolo[1,5-a]pyrimidin sowie ihre physiologisch verträglichen Salze und ihre tautomeren Formen, wenn ein tautomeres Gleichgewicht vorhanden ist. Bevorzugt ist 2,3-Diamino-6,7-dihydro-1H,5H-pyrazolo[1,2-a]pyrazol-1-on.

Besonders bevorzugte Entwicklerkomponenten werden ausgewählt aus mindestens einer Verbindung aus der Gruppe, die gebildet wird aus p-Phenylendiamin, p-Toluylendiamin, 2-(2-Hydroxyethyl)-p-phenylendiamin, 2-(1,2-Dihydroxyethyl)-p-phenylendiamin, N,N-Bis-(2-hydroxyethyl)-p-phenylendiamin, 2-Methoxymethyl-p-phenylendiamin, N-(4-Amino-3-methylphenyl)-N-[3-(1H-imidazol-1-yl)propyl]amin, N,N'-Bis-(2-hydroxyethyl)-N,N'-bis-(4-aminophenyl)-1,3-diamino-propan-2-ol, Bis-(2-hydroxy-5-aminophenyl)methan, 1,3-Bis-(2,5-diaminophenoxy)propan-2-ol, N,N'-Bis-(4-aminophenyl)-1,4-diazacycloheptan, 1,10-Bis-(2,5-diaminophenyl)-1,4,7,10-tetraoxadecan, p-Aminophenol, 4-Amino-3-methylphenol, 4-Amino-2-aminomethylphenol, 4-Amino-2-(1,2-dihydroxy-ethyl)phenol, 4-Amino-2-(diethylaminomethyl)phenol, 4,5-Diamino-1-(2-hydroxyethyl)pyrazol, 2,4,5,6-Tetraaminopyrimidin, 4-Hydroxy-2,5,6-triaminopyrimidin, 2-Hydroxy-4,5,6-triaminopyrimidin, 2,3-Diamino-6,7-dihydro-1H,5H-pyrazolo[1,2-a]pyrazol-1-on sowie deren physiologisch verträglichen Salzen. Ganz besonders bevorzugte Entwicklerkomponenten sind p-Toluylendiamin, 2-(2-Hydroxyethyl)-p-phenylendiamin, 2-Methoxymethyl-p-phenylendiamin, N-(4-Amino-3-methylphenyl)-N-[3-(1H-imidazol-1-yl)propyl]amin, und/oder 4,5-Diamino-1-(2-hydroxyethyl)-pyrazol sowie deren physiologisch verträglichen Salze.

Die Entwicklerkomponenten werden bevorzugt in einer Menge von 0,0001 bis 0,5 Gew.-%, vorzugsweise 0,001 bis 0,2 Gew.-%, jeweils bezogen auf das anwendungsbereite Mittel, verwendet.

Kupplerkomponenten bilden im Rahmen der oxidativen Färbung allein keine signifikante Färbung aus, sondern benötigen stets die Gegenwart von Entwicklerkomponenten. Daher ist es erfindungsgemäß bevorzugt, dass bei Verwendung mindestens einer Kupplerkomponente zusätzlich mindestens eine Entwicklerkomponente zum Einsatz kommt. Kupplerkomponenten im Sinne der Erfindung erlauben mindestens eine Substitution eines chemischen Restes des Kupplers durch die oxidierte Form der Entwicklerkomponente. Dabei bildet sich eine kovalente Bindung zwischen Kuppler- und Entwicklerkomponente aus.

Erfindungsgemäße Kupplerkomponenten werden bevorzugt als mindestens eine Verbindung aus einer der folgenden Klassen ausgewählt: m-Aminophenol, o-Aminophenol, m-Diaminobenzol, o-Diaminobenzol und/oder deren Derivate; Naphthalinderivate mit mindestens einer Hydroxygruppe; Dibeziehungsweise Trihydroxybenzol; Pyridinderivate; Pyrimidinderivate; bestimmte Indol-Derivate und Indolin-Derivate; Pyrazolonderivate (beispielsweise 1-Phenyl-3-methylpyrazol-5-on); Morpholinderivate (beispielsweise 6-Hydroxybenzomorpholin oder 6-Aminobenzomorpholin); Chinoxalinderivate (beispielsweise 6-Methyl-1,2,3,4-tetrahydrochinoxalin), sowie Gemische aus zwei oder mehreren Verbindungen aus einer oder mehreren dieser Klassen.

Bevorzugte m-Aminophenol-Kupplerkomponenten werden ausgewählt aus mindestens einer Verbindung aus der Gruppe, die gebildet wird aus 3-Aminophenol, 5-Amino-2-methylphenol, N-Cyclopentyl-3-aminophenol, 3-Amino-2-chlor-6-methylphenol, 2-Hydroxy-4-aminophenoxyethanol, 2,6-Dimethyl-3-aminophenol, 3-Trifluoroacetylamino-2-chlor-6-methylphenol, 5-Amino-4-chlor-2-methylphenol, 5-Amino-4-methoxy-2-methylphenol, 5-(2'-Hydroxyethyl)amino-2-methylphenol, 3-Diethylaminophenol, N-Cyclopentyl-3-aminophenol, 1,3-Dihydroxy-5-(methylamino)benzol, 3-Ethyl-amino-4-methylphenol, 2,4-Dichlor-3-aminophenol und deren physiologisch verträglichen Salzen.

Bevorzugte m-Diaminobenzol-Kupplerkomponenten werden ausgewählt aus mindestens einer Verbindung aus der Gruppe, die gebildet wird aus m-Phenylendiamin, 2-(2,4-Diaminophenoxy)ethanol, 1,3-Bis(2,4-diaminophenoxy)propan, 1-Methoxy-2-amino-4-(2'-hydroxyethylamino)benzol, 1,3-Bis-(2,4-diaminophenyl)propan, 2,6-Bis(2'-hydroxyethylamino)-1-methylbenzol, 2-({3-[(2-Hydroxyethyl)-arnino]-4-methoxy-5-methylphenyl}amino)ethanol, 2-({3-[(2-Hydroxyethyl)amino]-2-methoxy-5-methylphenyl}amino)ethanol, 2-({3-[(2-Hydroxyethyl)amino]-4,5-dimethylphenyl}amino)ethanol, 2-[3-Morpholin-4-ylphenyl)amino]ethanol, 3-Amino-4-(2-methoxyethoxy)-5-methylphenylamin, 1-Amino-3-bis-(2'-hydroxyethyl)aminobenzol und deren physiologisch verträglichen Salzen.

Bevorzugte o-Diaminobenzol-Kupplerkomponenten werden ausgewählt aus mindestens einer Verbindung aus der Gruppe, die gebildet wird aus 3,4-Diaminobenzoesäure und 2,3-Diamino-1-methylbenzol und deren physiologisch verträglichen Salzen.

Bevorzugte Naphthalinderivate mit mindestens einer Hydroxygruppe werden ausgewählt aus mindestens einer Verbindung der Gruppe, die gebildet wird aus 1-Naphthol, 2-Methyl-1-naphthol, 2-Hydroxymethyl-1-naphthol, 2-Hydroxyethyl-1-naphthol, 1,3-Dihydroxynaphthalin, 1,5-Dihydroxynaphthalin, 1,6-Dihydroxynaphthalin, 1,7-Dihydroxynaphthalin, 1,8-Dihydroxynaphthalin, 2,7-Dihydroxynaphthalin und 2,3-Dihydroxynaphthalin.

Bevorzugte Di- beziehungsweise Trihydroxybenzole und deren Derivate werden ausgewählt aus mindestens einer Verbindung der Gruppe, die gebildet wird aus Resorcin, Resorcinmonomethylether, 2-Methylresorcin, 5-Methylresorcin, 2,5-Dimethylresorcin, 2-Chlorresorcin, 4-Chlorresorcin, Pyrogallol und 1,2,4-Trihydroxybenzol.

Bevorzugte Pyridinderivate werden ausgewählt aus mindestens einer Verbindung der Gruppe, die gebildet wird aus 2,6-Dihydroxypyridin, 2-Amino-3-hydroxypyridin, 2-Amino-5-chlor-3-hydroxypyridin, 3-Amino-2-methylamino-6-methoxypyridin, 2,6-Dihydroxy-3,4-dimethylpyridin, 2,6-Dihydroxy-4-methylpyridin, 2,6-Diaminopyridin, 2,3-Diamino-6-methoxypyridin, 3,5-Diamino-2,6-dimethoxypyridin, 3,4-Diaminopyridin, 2-(2-Methoxyethyl)amino-3-amino-6-methoxypyridin, 2-(4'-Methoxyphenyl)amino-3-aminopyridin, und deren physiologisch verträglichen Salzen.

Bevorzugte Pyrimidinderivate werden ausgewählt aus mindestens einer Verbindung der Gruppe, die gebildet wird aus 4,6-Diaminopyrimidin, 4-Amino-2,6-dihydroxypyrimidin, 2,4-Diamino-6-hydroxypyrimidin, 2,4,6-Trihydroxypyrimidin, 2-Amino-4-methylpyrimidin, 2-Amino-4-hydroxy-6-methylpyrimidin und 4,6-Dihydroxy-2-methylpyrimidin und deren physiologisch verträglichen Salzen.

Bevorzugte Indolderivate werden ausgewählt aus mindestens einer Verbindung der Gruppe, die gebildet wird aus 4-Hydroxyindol, 6-Hydroxyindol und 7-Hydroxyindol und deren physiologisch verträglichen Salzen.

Bevorzugte Indolinderivate werden ausgewählt aus mindestens einer Verbindung der Gruppe, die gebildet wird aus 4-Hydroxyindolin, 6-Hydroxyindolin und 7-Hydroxyindolin und deren physiologisch verträglichen Salzen.
Erfindungsgemäß besonders bevorzugte Kupplerkomponenten werden ausgewählt unter 3-Aminophenol, 5-Amino-2-methylphenol, 3-Amino-2-chlor-6-methylphenol, 2-Hydroxy-4-aminophenoxy-ethanol, 5-Amino-4-chlor-2-methylphenol, 5-(2-Hydroxyethyl)-amino-2-methylphenol, 2,4-Dichlor-3-aminophenol, 2-Aminophenol, 3-Phenylendiamin, 2-(2,4-Diaminophenoxy)ethanol, 1,3-Bis(2,4-diaminophenoxy)propan, 1-Methoxy-2-amino-4-(2-hydroxyethylamino)benzol, 1,3-Bis(2,4-diamino-phenyl)propan, 2,6-Bis(2'-hydroxyethylamino)-1-methylbenzol, 2-({3-[(2-Hydroxyethyl)amino]-4-methoxy-5-methylphenyl}amino)ethanol, 2-({3-[(2-Hydroxyethyl)amino]-2-methoxy-5-methylphenyl}amino)ethanol, 2-({3-[(2-Hydroxyethyl)amino]-4,5-dimethylphenyl}amino)ethanol, 2-[3-Morpholin-4-ylphenyl)amino]ethanol, 3-Amino-4-(2-methoxyethoxy)-5-methylphenylamin, 1-Amino-3-bis-(2-hydroxyethyl)aminobenzol, Resorcin, 2-Methylresorcin, 4-Chlorresorcin, 1,2,4-Trihydroxybenzol, 2-Amino-3-hydroxypyridin, 3-Amino-2-methylamino-6-methoxypyridin, 2,6-Dihydroxy-3,4-dimethylpyridin, 3,5-Diamino-2,6-dimethoxypyridin, 1-Phenyl-3-methylpyrazol-5-on, 1-Naphthol, 1,5-Dihydroxynaphthalin, 2,7-Dihydroxynaphthalin, 1,7-Dihydroxynaphthalin, 1,8-Dihydroxynaphthalin, 4-Hydroxyindol, 6-Hydroxyindol, 7-Hydroxyindol, 4-Hydroxyindolin, 6-Hydroxyindolin, 7-Hydroxyindolin oder Gemischen dieser Verbindungen oder deren physiologisch verträglichen Salzen. Ganz besonders bevorzugt sind Resorcin, 2-Methylresorcin, 5-Amino-2-methylphenol, 3-Aminophenol, 2-(2,4-Diaminophenoxy)ethanol, 1,3-Bis(2,4-diaminophenoxy)propan, 1-Methoxy-2-amino-4-(2'-hydroxyethylamino)benzol, 2-Amino-3-hydroxypyridin und 1-Naphthol sowie eines deren physiologisch verträglichen Salze.

Die Kupplerkomponenten werden bevorzugt in einer Menge von 0,0001 bis 0,5 Gew.-%, vorzugsweise 0,001 bis 0,2 Gew.-%, jeweils bezogen auf das anwendungsbereite Mittel, verwendet.

Dabei werden Entwicklerkomponenten und Kupplerkomponenten im Allgemeinen in etwa molaren Mengen zueinander eingesetzt. Wenn sich auch der molare Einsatz als zweckmäßig erwiesen hat, so ist ein gewisser Überschuss einzelner Oxidationsfarbstoffvorprodukte nicht nachteilig, so dass Entwicklerkomponenten und Kupplerkomponenten in einem Mol-Verhältnis von 1 zu 0,5 bis 1 zu 3, insbesondere 1 zu 1 bis 1 zu 2, stehen können.

Die erfindungsgemäßen Zubereitungen (M1) enthalten mindestens einen direktziehenden Farbstoff. Dabei handelt sich um Farbstoffe, die direkt auf das Haar aufziehen und keinen oxidativen Prozess zur Ausbildung der Farbe benötigen. Direktziehende Farbstoffe können in anionische, kationische und nichtionische direktziehende Farbstoffe unterteilt werden. Üblicherweise sind es Nitrophenylendiamine, Nitroaminophenole, Azofarbstoffe, Anthrachinone oder Indophenole. Die direktziehenden Farbstoffe werden jeweils bevorzugt in einer Menge von 0,0001 bis 5,0 Gew.-%, bevorzugt von 0,001 bis 1,5 Gew.-%, jeweils bezogen auf die gesamte Anwendungszubereitung, eingesetzt. Die Gesamtmenge an direktziehenden Farbstoffen beträgt vorzugsweise höchstens 1,0 Gew.-%.

Bevorzugte anionische direktziehende Farbstoffe sind die unter den internationalen Bezeichnungen bzw. Handelsnamen Acid Yellow 1, Yellow 10, Acid Yellow 23, Acid Yellow 36, Acid Orange 7, Acid Red 33, Acid Red 52, Pigment Red 57:1, Acid Blue 7, Acid Green 50, Acid Violet 43, Acid Black 1, Acid Black 52, Bromphenolblau und Tetrabromphenolblau bekannten Verbindungen. Bevorzugte kationische direktziehende Farbstoffe sind kationische Triphenylmethanfarbstoffe, wie Basic Blue 7, Basic Blue 26, Basic Violet 2 und Basic Violet 14, aromatischen Systeme, die mit einer quaternären Stickstoffgruppe substituiert sind, wie beispielsweise Basic Yellow 57, Basic Red 76, Basic Blue 99, Basic Brown 16 und Basic Brown 17, sowie direktziehende Farbstoffe, die einen Heterocyclus enthalten, der mindestens ein quaternäres Stickstoffatom aufweist, insbesondere Basic Yellow 87, Basic Orange 31 und Basic Red 51. Die kationischen direktziehenden Farbstoffe, die unter dem Warenzeichen Arianor^{®} vertrieben werden, sind erfindungsgemäß ebenfalls besonders bevorzugte kationische direktziehende Farbstoffe. Als nichtionische direktziehende Farbstoffe eignen sich insbesondere nichtionische Nitro- und Chinonfarbstoffe und neutrale Azofarbstoffe. Bevorzugte nichtionische direktziehende Farbstoffe sind die unter den internationalen Bezeichnungen bzw. Handelsnamen HC Yellow 2, HC Yellow 4, HC Yellow 5, HC Yellow 6, HC Yellow 12, HC Orange 1,

Disperse Orange 3, HC Red 1, HC Red 3, HC Red 10, HC Red 11, HC Red 13, HC Red BN, HC Blue 2, HC Blue 11, HC Blue 12, Disperse Blue 3, HC Violet 1, Disperse Violet 1, Disperse Violet 4, Disperse Black 9 bekannten Verbindungen, sowie 1,4-Diamino-2-nitrobenzol, 2-Amino-4-nitrophenol, 1,4-Bis-(2-hydroxyethyl)-amino-2-nitrobenzol, 3-Nitro-4-(2-hydroxyethyl)aminophenol, 2-(2-Hydroxyethyl)amino-4,6-dinitrophenol, 4-[(2-Hydroxyethyl)amino]-3-nitro-1-methylbenzol, 1-Amino-4-(2-hydroxyethyl)amino-5-chlor-2-nitrobenzol, 4-Amino-3-nitrophenol, 1-(2'-Ureidoethyl)amino-4-nitrobenzol, 2-[(4-Amino-2-nitrophenyl)amino]-benzoesäure, 6-Nitro-1,2,3,4-tetrahydrochinoxalin, 2-Hydroxy-1,4-naphthochinon, Pikraminsäure und deren Salze, 2-Amino-6-chloro-4-nitrophenol, 4-Ethylamino-3-nitrobenzoesäure und 2-Chlor-6-ethylamino-4-nitrophenol. Erfindungsgemäß bevorzugte Farbstoffkombinationen sind solche, die mindestens die Kombination aus Tetrabromphenolblau, und Acid Red 92; Tetrabromphenolblau und Acid Red 98; Tetrabromphenolblau und Acid Red 94; Tetrabromphenolblau und Acid Red 87 oder Tetrabromphenolblau und Acid Red 51.

Zur Einstellung eines basischen pH-Werts der Anwendungsmischung und insbesondere zur Stabilisierung von Farbstoffvorprodukten enthält die erfindungsgemäße Zubereitung (M1) zusätzlich mindestens ein Alkalisierungsmittel, ausgewählt aus Ammoniak, Natriumhydroxid, Natriumcarbonat, Kaliumhydroxid, Kaliumcarbonat, Natriumhydrogencarbonat, Ammoniumcarbonat, Ammoniumhydrogencarbonat, Arginin, Histidin, Monoethanolamin und/oder 2-Amino-2-methylpropanol.

Erfindungsgemäß einsetzbare, organische Alkalisierungsmittel werden bevorzugt ausgewählt aus Alkanolaminen aus primären, sekundären oder tertiären Aminen mit einem C₂-C₆-Alkylgrundkörper, der mindestens eine Hydroxylgruppe.trägt. Erfindungsgemäß ganz besonders bevorzugte Alkanolamine werden ausgewählt aus der Gruppe 2-Aminoethan-1-ol (Monoethanolamin), 2-Amino-2-methylpropan-1-ol und 2-Amino-2-methyl-propan-1,3-diol. Ein bevorzugtes Alkanolamin ist Monoethanolamin. Geeignete basische Aminsäuren sind Lysin, Arginin und Ornithin. Das erfindungsgemäße, anorganische Alkalisierungsmittel sind bevorzugt ausgewählt aus der Gruppe, die gebildet wird aus Natriumhydroxid, Kaliumhydroxid, Calciumhydroxid, Bariumhydroxid, Natriumphosphat, Kaliumphosphat, Natriumsilicat, Kaliumsilicat, Ammoniumhydrogencarbonat, Natriumhydrogencarbonat, Kaliumhydrogencarbonat, Ammoniumcarbonat, Natriumcarbonat und Kaliumcarbonat.

Die Oxidationsmittelzubereitung (M2) enthält in einem wässrigen, kosmetischen Träger als chemisches Oxidationsmittel mindestens Wasserstoffperoxid. Wasserstoffperoxid wird dabei entweder als vorzugsweise wässrige Lösung oder in Form einer festen Anlagerungsverbindung von Wasserstoffperoxid an anorganische oder organische Verbindungen, wie beispielsweise Natriumperborat, Natriumpercarbonat, Magnesiumpercarbonat, Natriumpercarbamid, Polyvinylpyrrolidinon·n H₂O₂ (n ist eine positive ganze Zahl größer 0), Harnstoffperoxid und Melaminperoxid, eingesetzt. Erfindungsgemäß bevorzugte wässrige Phasen (I) enthalten wässrige Wasserstoffperoxid-Lösungen. Die Konzentration einer Wasserstoffperoxid-Lösung wird einerseits von den gesetzlichen Vorgaben und andererseits von dem gewünschten Effekt bestimmt. Vorzugsweise werden 3 Gew.-%ige bis 12 Gew.-%ige Lösungen von Wasserstoffperoxid in Wasser verwendet. Die erfindungsgemäßen, anwendungsbereiten Mittel zur Farbveränderung keratinischer Fasern enthalten besonders bevorzugt 0,5 bis 15 Gew.-%, vorzugsweise 1 bis 12 Gew.-%, besonders bevorzugt 1,5 bis 9 Gew.-% und insbesondere 2 bis 8 Gew.-% Wasserstoffperoxid (berechnet als 100%iges H₂O₂), jeweils berechnet auf das Gesamtgewicht der Mittel.
Die Oxidationszubereitung (M2) enthält weiterhin mindestens ein anionisches, polymeres Verdickungsmittel, welches ausgewählt ist aus vernetzten Polyacrylsäuren , die ein Molekulargewicht MW von mindestens 500000 g/mol besitzen, enthält, wobei die Polyacrylsäuren Polymerisate aus Acrylsäure selbst darstellen.

Unter vernetzten Polyacrylsäure-Polymeren sind solche Polymerisate zu verstehen, denen zur Vernetzung während der Polymerisation ein gewisser Anteil an bi- oder multifunktionalen Monomeren zugesetzt wurde. Bevorzugte Vernetzungsmittel sind dabei Ethylenglycoldimethacrylat, 1,9-Decadien, Divinylbenzol sowie Allylether von Pentaerythrit, von Sucrose, von Ethylenglycol und von Propylenglycol. Geeignete polymere Verbindungen sind beispielsweise unter dem Warenzeichnen Carbopol^{®} im Handel erhältlich und unter der INCI-Bezeichnung Carbomer bekannt.

Ein erfindungsgemäß besonders bevorzugtes anionisches, polymeres Verdickungsmittel wird unter dem Handelsnamen Carbomer 954 von der Firma Lubrizol (Noveon) vertrieben.

Bevorzugt enthält die erfindungsgemäße Oxidationsmittelzubereitung (M2) das oder die anionische(n), polymere(n) Verdickungsmittel zu 0,01 bis 20 Gew.-%, bevorzugt zu 0,1 bis 5 Gew.-%, jeweils bezogen auf das Gesamtgewicht der Zubereitung (M2).

Die erfindungsgemäße Oxidationsmittelzubereitung (M2) besitzt erfindungsgemäß bevorzugt einen schwach sauren pH-Wert, bevorzugt einen pH-Wert von pH 2 bis pH 6, besonders bevorzugt von pH 2,5 bis pH 4,5. Bei den pH-Werten im Sinne der vorliegenden Erfindung handelt es sich um pH-Werte, die bei einer Temperatur von 22°C gemessen wurden. Zur Einstellung des pH-Werts sind dem Fachmann gängige Acidifizierungs- und Alkalisierungsmittel geläufig. Erfindungsgemäß bevorzugte Acidifizierungsmittel sind Genuss-Säuren, wie beispielsweise Milchsäure, Zitronensäure, Essigsäure, Äpfelsäure oder Weinsäure, sowie verdünnte Mineralsäuren.

Es kann ist erfindungsgemäß bevorzugt sein, wenn die Zubereitung (M2) weiterhin nur einen geringen Anteil an grenzflächenaktiven, insbesondere anionischen grenzflächenaktiven Substanzen enthält. Eine Ausführungsform der vorliegenden Erfindung ist daher dadurch gekennzeichnet, dass die Zubereitung (M2) zusätzlich mindestens ein anionisches Tensid in einem Gewichtsanteil von 0,05 bis 1,5 Gew.-%, bezogen auf das Gesamtgewicht der Zubereitung (M2), enthält.

Als grenzflächenaktive Substanzen im Sinne der Erfindung gelten dabei Emulgatoren und Tenside. Grenzflächenaktive Substanzen zeichnen sich durch hydrophobe und hydrophile Strukturmerkmale aus und ermöglichen so eine Durchmischung der Phasen unter Ausbildung von Micellen und stabilen Emulsionen. Anionische Tenside im Sinne der Erfindung sind alle für die Verwendung am menschlichen Körper geeigneten anionischen oberflächenaktiven Stoffe. Diese sind gekennzeichnet durch eine wasserlöslich machende, anionische Gruppe wie beispielsweise eine Carboxylat-, Sulfat-, Sulfonat- oder Phosphat-Gruppe und eine lipophile Alkylgruppe mit etwa 8 bis 30 C-Atomen. Zusätzlich können im Molekül Glykol- oder Polyglykolether-Gruppen, Ester-, Ether- und Amidgruppen sowie Hydroxylgruppen enthalten sein. Beispiele für solche anionischen Tenside sind, jeweils in Form der Natrium-, Kalium- und Ammonium- sowie der Mono, Di- und Trialkanolammoniumsalze mit 2 bis 4 C-Atomen in der Alkanolgruppe, lineare und verzweigte Fettsäuren mit 8 bis 30 C-Atomen (Seifen); Ethercarbonsäuren, insbesondere der Formel RO(CH₂CH₂O)ₓCH₂COOH, in der R eine lineare Alkylgruppe mit 8 bis 30 C-Atomen und x = 0 oder 1 bis 16 ist; Acylsarcoside; Acyltauride; Acylisethionate; Sulfobernsteinsäuremono- und -dialkylester sowie Sulfobernsteinsäuremono-alkylpolyoxyethylester; lineare Alkansulfonate; lineare α-Olefinsulfonate; Sulfonate ungesättigter Fettsäuren; α-Sulfofettsäuremethylester von Fettsäuren; Alkylsulfate und Alkylethersulfate, insbesondere der Formel RO(CH₂CH₂O)ₓSO₃H, in der R für eine lineare Alkylgruppe mit 8 bis 30 C-Atomen und x für 0 oder eine Zahl von 1 bis 12 steht; Gemische oberflächenaktiver Hydroxysulfonate; sulfatierte Hydroxyalkylpolyethylen- und/oder Hydroxyalkylenpropylenglykolether; Ester der Weinsäure und Zitronensäure mit Alkoholen; Alkyl- und/oder Alkenyletherphosphate der Formel RO(C₂H₄O)ₓP(=O)(OH)(OR'), worin R für einen aliphatischen, gegebenenfalls ungesättigten Kohlenwasserstoffrest mit 8 bis 30 Kohlenstoffatomen, R' für Wasserstoff, einen Rest (CH₂CH₂O)_{y}R und x und y unabhängig voneinander für eine Zahl von 1 bis 10 steht; sulfatierte Fettsäurealkylenglykolester der Formel RC(O)O(alkO)ₙSO₃H, in der R für einen linearen oder verzweigten, aliphatischen, gesättigten und/oder ungesättigten Alkylrest mit 6 bis 22 C-Atomen, alk für CH₂CH₂, CHCH₃CH₂ und/oder CH₂CHCH₃ und n für eine Zahl von 0,5 bis 5 steht; sowie Monoglyceridsulfate und Monoglyceridethersulfate.

Erfindungsgemäß kann die Oxidationsmittelzubereitung zusammen mit einem Katalysator auf das Haar aufgebracht werden, der die Oxidation der Farbstoffvorprodukte zusätzlich aktiviert. Solche Katalysatoren sind z. B. bestimmte Enzyme, Iodide, Chinone oder Metallionen. Hierfür geeignete Enzyme sind z. B. Peroxidasen, die die Wirkung geringer Mengen an Wasserstoffperoxid deutlich verstärken können. Ein Einsatz bestimmter Metallionen oder -komplexe kann ebenfalls bevorzugt sein. Geeignete Metallionen sind beispielsweise Zn²⁺, Cu²⁺, Fe²⁺, Fe³⁺, Mn²⁺, Mn⁴⁺, Li⁺, Mg²⁺, Ca²⁺, Ce⁴⁺, V³⁺, Co²⁺, Ru³⁺ und Al³⁺. Besonders geeignet sind dabei Zu²⁺, Cm²⁺ und Mn²⁺.

Weiterhin hat es sich als vorteilhaft erweisen, wenn die Oxidationsmittelzubereitungen mindestens einen Stabilisator oder Komplexbildner enthalten. Besonders bevorzugte Stabilisatoren sind Phenacetin, Alkalibenzoate (Natriumbenzoat) und Salicylsäure.

Erfindungsgemäß bevorzugt ist auch der Einsatz von sogenannten Komplexbildnern. Komplexbilder sind Stoffe, die Metallionen komplexieren können. Bevorzugte Komplexbildner sind sogenannte Chelatkomplexbildner, also Stoffe, die mit Metallionen cyclische Verbindungen bilden, wobei ein einzelner Ligand mehr als eine Koordinationsstelle an einem Zentralatom besetzt, d. h. mindestens "zweizähnig" ist. Gebräuchliche und im Rahmen der vorliegenden Erfindung bevorzugte Chelatkomplexbildner sind beispielsweise Polyoxycarbonsäuren, Polyamine, Ethylendiamintetraessigsäure (EDTA), Nitrilotriessigsäure (NTA) und Hydroxyethandiphosphonsäuren bzw. deren Alkalisalze. Auch komplexbildende Polymere, also Polymere, die entweder in der Hauptkette selbst oder seitenständig zu dieser funktionelle Gruppen tragen, die als Liganden wirken können und mit geeigneten Metall-atomen in der Regel unter Bildung von Chelat-Komplexen reagieren, sind erfindungsgemäß einsetzbar. Die Polymer-gebundenen Liganden der entstehenden Metall-Komplexe können dabei aus nur einem Makromolekül stammen oder aber zu verschiedenen Polymerketten gehören. Erfindungsgemäß bevorzugte Komplexbildner sind stickstoffhaltigen Polycarbonsäuren, insbesondere EDTA, und Phosphonate, vorzugsweise Hydroxyalkan- bzw. Aminoalkanphosphonate und insbesondere 1-Hydroxyethan-1,1-diphosphonat(HEDP) bzw. dessen Di-oder Tetranatriumsalz und/ oder Ethylendiamintetramethylenphosphonat (EDTMP) bzw. dessen Hexanatriumsalz und/oder Diethylentriaminpentamethylenphosphonat (DTPMP) bzw. dessen Hepta- oder Octanatriumsalz.

Erfindungsgemäße, anwendungsbereite Mittel sind wässrige, fließfähige Zubereitungen. Die erfindungsgemäßen Mittel können weiterhin alle für solche Zubereitungen bekannten Wirk-, Zusatz- und Hilfsstoffe enthalten. Die anwendungsbereiten Mittel als Mischung aus Mittel (M1) und (M2) können dabei oberflächenaktive Substanzen, ausgewählt aus den oben angeführten anionischen sowie nichtionischen, zwitterionischen, amphoteren und kationischen Tensiden enthalten.

Als zwitterionische Tenside werden solche oberflächenaktiven Verbindungen bezeichnet, die im Molekül mindestens eine quartäre Ammoniumgruppe und mindestens eine Carboxylat-, Sulfonat- oder Sulfat-Gruppe tragen. Beispiele solcher zwitterionischen Tenside sind die sogenannten Betaine wie die N-Alkyl-N,N-dimethylammonium-glycinate, beispielsweise das Kokosalkyl-dimethylammoniumglycinat, N-Acyl-aminopropyl-N,N-dimethylammoniumglycinate, beispielsweise das Kokosacylaminopropyl-dimethylammoniumglycinat, und 2-Alkyl-3-carboxymethyl-3-hydroxyethyl-imidazoline mit jeweils 8 bis 18 C-Atomen in der Alkyl- oder Acylgruppe sowie das Kokosacylaminoethylhydroxyethylcarboxymethylglycinat. Ein bevorzugtes zwitterionisches Tensid ist das unter der INCI-Bezeichnung Cocamidopropyl Betaine bekannte Fettsäureamid-Derivat.

Unter amphoteren Tensiden werden solche oberflächenaktiven Verbindungen verstanden, die außer einer C₈-C₂₄-Alkyl- oder -Acylgruppe im Molekül mindestens eine freie Aminogruppe und mindestens eine -COOH- oder -SO₃H-Gruppe enthalten und zur Ausbildung innerer Salze befähigt sind. Übliche amphotere Tenside sind N-Alkylglycine, N-Alkylpropionsäuren, N-Alkylaminobuttersäuren, N-Alkyliminodipropionsäuren, N-Hydroxyethyl-N-alkylamidopropylglycine, N-Alkyltaurine, N-Alkylsarcosine, 2-Alkylaminopropionsäuren und Alkylaminoessigsäuren mit jeweils etwa 8 bis 24 C-Atomen in der Alkylgruppe. Beispielhafte amphotere Tenside sind N-Kokosalkylaminopropionat, Kokosacylaminoethylaminopropionat und C₁₂-C₁₈-Acylsarcosin.

Nichtionische Tenside und Emulgatoren enthalten als hydrophile Gruppe z. B. eine Polyolgruppe, eine Polyalkylenglykolethergruppe oder eine Kombination aus Polyol- und Polyglykolethergruppe. Solche Verbindungen sind beispielsweise Anlagerungsprodukte von 1 bis 50 Mol Ethylenoxid und/oder 0 bis 5 Mol Propylenoxid an lineare und verzweigte Fettalkohole mit 8 bis 30 C-Atomen, an Fettsäuren mit 8 bis 30 C-Atomen und an Alkylphenole mit 8 bis 15 C-Atomen in der Alkylgruppe; mit einem Methyl- oder C₂-C₆-Alkylrest endgruppenverschlossene Anlagerungsprodukte von 1 bis 50 Mol Ethylenoxid und/oder 0 bis 5 Mol Propylenoxid an lineare und verzweigte Fettalkohole mit 8 bis 30 C-Atomen, an Fettsäuren mit 8 bis 30 C-Atomen und an Alkylphenole mit 8 bis 15 C-Atomen in der Alkylgruppe, wie beispielsweise die unter den Verkaufsbezeichnungen Dehydol LS, Dehydol LT (Cognis) erhältlichen Typen; Polyglycerinester und alkoxylierte Polyglycerinester, wie beispielsweise Poly(3)glycerindiisostearat (Handelsprodukt: Lameform TGI (Henkel)) und Poly(2)glycerinpolyhydroxy-stearat (Handelsprodukt: Dehymuls PGPH (Henkel)); Polyolfettsäureester, wie beispielsweise das Handelsprodukt Hydagen HSP (Cognis) oder Sovermol-Typen (Cognis); höher alkoxylierte, propoxylierte und insbesondere ethoxylierte, Mono-, Di- und Triglyceride mit Alkoxylierungsgrad von größer als 5, wie beispielsweise Glycerinmonolaurat + 20 Ethylenoxid und Glycerinmonostearat + 20 Ethylenoxid; Aminoxide; Hydroxymischether; Sorbitanfettsäureester und Anlagerungeprodukte von Ethylenoxid an Sorbitanfettsäureester wie beispielsweise die Polysorbate und Sorbitanmonolaurat + 20 Mol Ethylenoxid (EO); Zuckerfettsäureester und Anlagerungsprodukte von Ethylenoxid an Zuckerfettsäureester; Anlagerungsprodukte von Ethylenoxid an Fettsäurealkanolamide und Fettamine; Fettsäure-N-alkylglucamide; Alkylphenole und Alkylphenolalkoxylate mit 6 bis 21, insbesondere 6 bis 15 Kohlenstoffatomen in der Alkylkette und 5 bis 30 Ethylenoxid- und/oder Propylenoxid-Einheiten; Alkylpolyglykoside entsprechend der allgemeinen Formel RO-(Z)ₓ, wobei R für Alkyl, Z für Zucker sowie x für die Anzahl der Zuckereinheiten steht.

Zu den nichtionischen Emulgatoren im Sinne der Erfindung zählen weiterhin die Polymerisationsprodukte aus Ethylenoxid und Propylenoxid an gesättigte oder ungesättigte Fettalkohole; Fettsäureester mehrwertiger Alkohole mit gesättigten oder ungesättigten Fettsäuren; Alkylester von gesättigten oder ungesättigten Fettsäuren oder Alkylphenole und deren Alkoxylate; insbesondere Ethylenglykolether von Fettalkoholen; gemischte Ethylen- und Propylenglykolether mit Fettalkoholen; Fettsäureester an Sorbitan sowie Polyethylenglykol; Ester von nicht hydroxylierten C₆-C₃₀-Alkylmonocarbonäuren mit Polyethylenglykol; und Anlagerungsprodukte von Alkylphenolen an Ethylen- und/oder Propylenoxid.

Erfindungsgemäß bevorzugt sind in anwendungsbereiten Mitteln kationische Tenside vom Typ der quartären Ammoniumverbindungen, der Esterquats und der Amidoamine. Bevorzugte quaternäre Ammoniumverbindungen sind Ammoniumhalogenide, insbesondere Chloride und Bromide, wie Alkyltrimethylammoniumchloride, Dialkyldimethylammoniumchloride und Trialkylmethylammoniumchloride sowie die unter den INCI-Bezeichnungen Quaternium-27 und Quaternium-83 bekannten Imidazolium-Verbindungen. Weitere erfindungsgemäß verwendbare kationische Tenside stellen die quaternisierten Proteinhydrolysate dar. Alkylamidoamine werden üblicherweise durch Amidierung natürlicher oder synthetischer Fettsäuren und Fettsäureschnitte mit Dialkylaminoaminen hergestellt, wie Stearamidopropyl-dimethylamin. Ebenfalls bevorzugte Esterquats sind quaternierte Estersalze von Fettsäuren mit Triethanolamin, quaternierte Estersalze von Fettsäuren mit Diethanolalkylaminen und quaternierten Estersalzen von Fettsäuren mit 1,2-Dihydroxypropyldialkylaminen. Solche Produkte werden beispielsweise unter den Warenzeichen Stepantex, Dehyquart und Armocare vertrieben. Die Produkte Armocare VGH-70, ein N,N-Bis(2-Palmitoyloxyethyl)dimethyl-ammoniumchlorid, sowie Dehyquart F-75, Dehyquart C-4046, Dehyquart L80 und Dehyquart AU-35 sind Beispiele für solche Esterquats. Die kationischen Tenside sind in den erfindungsgemäß verwendeten Mitteln bevorzugt in Mengen von 0,05 bis 10 Gew.-%, bezogen auf das gesamte Mittel, enthalten. Mengen von 0,1 bis 5 Gew.-% sind besonders bevorzugt.

In einer bevorzugten Ausführungsform können nicht-ionische, zwitterionische und/oder amphotere Tenside sowie deren Mischungen bevorzugt sein.

Weitere, erfindungsgemäß einsetzbare Wirk-, Hilfs- und Zusatzstoffe sind beispielsweise nichtionische Polymere (wie Vinylpyrrolidinon/Vinylacrylat-Copolymere, Polyvinylpyrrolidinon und Vinylpyrrolidinon/Vinylacetat-Copolymere und Polysiloxane); zwitterionische und amphotere Polymere (wie Acrylamidopropyltrimethylammoniumchlorid/Acrylat-Copolymere und Octylacrylamid/Methylmethacrylat/tert-Butylaminoethylmethacrylat/2-Hydroxypropylmethacrylat-Copolymere); Verdickungsmittel (wie Agar-Agar, Guar-Gum, Alginate, Xanthan-Gum, Gummi arabicum, Karaya-Gummi, Johannisbrotkernmehl, Leinsamengummen, Dextrane, Cellulose-Derivate, z. B. Methylcellulose, Hydroxyalkylcellulose und Carboxymethylcellulose, Stärke-Fraktionen und Derivate wie Amylose, Amylopektin und Dextrine, Tone wie Bentonit oder vollsynthetische Hydrokolloide wie z.B. Polyvinylalkohol); Strukturanden (wie Zucker, Maleinsäure und Milchsäure) und Konsistenzgeber (wie Zuckerester, Polyolester oder Polyolalkylether); Proteinhydrolysate (insbesondere Elastin-, Kollagen-, Keratin-, Milcheiweiß-, Sojaprotein- und Weizenproteinhydrolysate, deren Kondensationsprodukte mit Fettsäuren); Parfümöle; Pflegeöle; Cyclodextrine; Entschäumer wie Silicone; Farbstoffe und Pigmente zum Anfärben des Mittels; Antischuppenwirkstoffe (wie Piroctone Olamine, Zink Omadine und Climbazol); Lichtschutzmittel (insbesondere derivatisierte Benzophenone, Zimtsäure-Derivate und Triazine); Wirkstoffe (wie Allantoin, Pyrrolidoncarbonsäuren, Cholesterin und deren Salze); weitere Fette und Wachse (wie Fettalkohole, Bienenwachs, Montanwachs und Paraffine); Quell- und Penetrationsstoffe (wie Glycerin, Propylenglykolmonoethylether, Carbonate, Hydrogencarbonate, Guanidine, Harnstoffe sowie primäre, sekundäre und tertiäre Phosphate); Trübungsmittel (wie Latex, Styrol/PVP- und Styrol/Acrylamid-Copolymere); Perlglanzmittel (wie Ethylenglykolmono- und -distearat sowie PEG-3-distearat); Treibmittel (wie Propan-Butan-Gemische, N₂O, Dimethylether, CO₂ und Luft) sowie Antioxidantien.

Die Auswahl dieser weiteren Stoffe wird der Fachmann gemäß der gewünschten Eigenschaften der Mittel treffen. Bezüglich weiterer fakultativer Komponenten sowie der eingesetzten Mengen dieser Komponenten wird ausdrücklich auf die dem Fachmann bekannten einschlägigen Handbücher, z. B. Kh. Schrader, Grundlagen und Rezepturen der Kosmetika, 2. Auflage, Hüthig Buch Verlag, Heidelberg, 1989, verwiesen.

Die gebrauchsfertigen Mittel aus Oxidationsmittelzubereitung (M2) und Farbveränderungsmittel (M1) weisen bevorzugt einen pH-Wert im Bereich von 6 bis 12 auf. Erfindungsgemäß bevorzugte Mittel sind dadurch gekennzeichnet, dass sie einen alkalischen pH-Wert besitzen. Eine weitere bevorzugte Ausführungsform der vorliegenden Erfindung besteht darin, dass das anwendungsbereite Mittel einen pH-Wert von 8,5 bis 11,5 bevorzugt 9,5 bis 11,5 besitzt. Bei den pH-Werten im Sinne der vorliegenden Erfindung handelt es sich um pH-Werte, die bei einer Temperatur von 22 °C gemessen wurden. Die zur Einstellung des pH-Werts gängigen Acidifizierungs- und Alkalisierungsmittel sind weiter oben ausgeführt.

Das erfindungsgemäße Mittel wird vor der Anwendung durch Vermischen der Farbveränderungszubereitung (M1) und der Oxidationszubereitung (M2) hergestellt. Es ist daher vorteilhaft, dem Anwender beide Zubereitungen in einem Set anzubieten. Daher ist eine bevorzugte Darreichungsform des anwendungsbereiten Mittels eine getrennte Verpackungseinheit, worin die Mittel (M1) und (M2) jeweils getrennt voneinander verpackt vorliegen.

Ein weiterer Gegenstand der vorliegenden Erfindung ist daher eine Verpackungseinheit (Kit-of-Parts), welche mindestens zwei voneinander getrennt konfektionierte Containern enthält, wobei ein erster Container (C1) eine Farbveränderungszubereitung (M1), enthaltend in einem kosmetischen Träger mindestens eine farbverändernde Komponente,
und ein zweiter Container (C2) eine Oxidationsmittelzubereitung (M2), enthaltend in einem wässrigen, kosmetischen Träger als chemisches Oxidationsmittel mindestens Wasserstoffperoxid und zusätzlich mindestens ein anionisches, polymeres Verdickungsmittel, ausgewählt aus vernetzten Polyacrylsäuren, enthält.

Unter Container wird im Rahmen der vorliegenden Erfindung eine Umhüllung verstanden, die in Form einer gegebenenfalls wieder-verschließbaren Flasche, einer Tube, einer Dose, eines Tütchens, eines Sachets oder ähnlichen Umhüllungen vorliegt. Dem Umhüllungsmaterial sind erfindungsgemäß keine Grenzen gesetzt. Bevorzugt handelt es sich jedoch dabei um Umhüllungen aus Glas oder Kunststoff.

Eine weitere Ausführungsform dieses Erfindungsgegenstands ist gegeben, wenn das Mittel (M1) aus Container (C1) die Färbezubereitung darstellt und als farbverändernde Komponente mindestens einen direktziehenden Farbstoff enthält.

Weiterhin kann es erfindungsgemäß besonders vorteilhaft sein, wenn das genannte Kit-of-Parts mindestens ein weiteres Haarbehandlungsmittel in einem getrennten Container enthält, insbesondere eine Konditioniermittelzubereitung. Darüber hinaus kann die Verpackungseinheit Applikationshilfen, wie Kämme, Bürsten oder Pinsel, persönliche Schutzkleidung, insbesondere Ein-Weg-Handschuhe, sowie gegebenenfalls eine Gebrauchsanleitung umfassen.

Hinsichtlich der bevorzugten Ausführungsführungsformen der Mittel (M1) und (M2) gelten mutatis mutandis die obigen Ausführungen der vorangehenden Erfindungsgegenstände.

Ein weiterer Erfindungsgegenstand ist ein Verfahren zur Farbveränderung keratinischer Fasern, insbesondere menschlicher Haare, welches dadurch gekennzeichnet ist, dass aus einer Mehrkomponentenverpackungseinheit gemäß dem vorangegangenen Erfindungsgegenstand die beiden Mittel (M1) und (M2) in einem der Container (C2) oder (C1) vereinigt werden, der wiederverschlossene Container daraufhin geschüttelt wird, und das im Container resultierende, anwendungsbereite Farbveränderungsmittel anschließend auf die Fasern aufgebracht wird, für eine Einwirkdauer von 5 bis 60 min auf den Fasern belassen und schließlich ausgespült wird.

Im Falle eines farbgebenden Mittels beträgt die bevorzugte Einwirkzeit 5 bis 40 min, bevorzugt 10 bis 30 min. Im Falle von aufhellenden oder bleichenden Farbveränderungsmitteln liegt die bevorzugte Einwirkzeit bei 30 bis 60 min, bevorzugt bei 40 bis 60 min. Die Anwendungstemperaturen können in einem Bereich zwischen 15 und 40 °C liegen. Nach der Einwirkungszeit wird das verbleibende Mittel durch Ausspülen von dem Haar entfernt. Das Nachwaschen mit einem Shampoo entfällt, wenn ein stark tensidhaltiger Träger verwendet wurde.

Zur verbesserten Durchmischung ist vorteilhaft, wenn der Container (C2), welcher die Oxidationsmittelzubereitung (M2) enthält, eine wieder-verschließbare Öffnung, wie beispielsweise einen Schnapp- oder einen Schraubverschluss, besitzt. Dies ermöglicht die erleichterte Zugabe des farbverändernden Mittels aus Container (C1), welcher seinerseits bevorzugt in Form eines Tütchens oder Sachets im Falle von wasserfreien, insbesondere pulverförmigen Farbveränderungsmitteln, oder in Form einer Tube im Falle von fließfähigen Farbveränderungsmitteln vorliegt. Es ist bevorzugt, die einzelnen Zubereitungen zu vermischen und das anwendungsbereite Mittel dann zeitnah auf die keratinischen Fasern zu applizieren.

Eine besondere Ausführungsform dieses Erfindungsgegenstands ist daher ein Verfahren zur Farbveränderung keratinischer Fasern, insbesondere menschlicher Haare, welches dadurch gekennzeichnet ist, dass aus einer Mehrkomponentenverpackungseinheit gemäß dem vorangegangenen Erfindungsgegenstand der Inhalt des Containers (C1) in den Container (C2) gefüllt wird, der wiederverschlossene Container (C2) daraufhin geschüttelt wird, und das im Container (C2) resultierende, anwendungsbereite Farbveränderungsmittel anschließend auf die Fasern aufgebracht wird, für eine Einwirkdauer von 5 bis 60 min auf den Fasern belassen und dann ausgespült wird.

Eine Ausführungsform des erfindungsgemäßen Verfahrens ist schließlich dadurch gekennzeichnet, dass das resultierende, anwendungsbereite Farbveränderungsmittel eine Viskosität von 5 bis 100 Pa·s, bevorzugt 10 bis 30 Pa·s und insbesondere bevorzugt 12 bis 25 Pa·s (Brookfield, 22°C, Spindel #5, 4 rpm), besitzt.

Im Rahmen dieses Gegenstandes der Erfindung gelten mutatis mutandis die oben getroffenen Aussagen analog.

Die folgenden Beispiele sollen den Gegenstand der vorliegenden Erfindung näher erläutern ohne diesen in irgendeiner Weise zu beschränken.

### Beispiele (nicht erfindungsgemäß)

### 1) Färbecreme FC (Tabelle 1; Mengenangaben in Gew.-%)

| | |
|---|---|
| Lanette D | 6,60 |
| Lorol C12-18 techn. | 2,40 |
| Eumulgin B2 | 0,60 |
| Eumulgin B1 | 0,60 |
| Akypo Soft 45HP | 10,00 |
| Protelan MST 35 | 6,00 |
| Texapon K 14 S Special, 70 % | 2,80 |
| Produkt W 37194 | 3,75 |
| Natriumsulfit, wasserfrei | 0,00 |
| Ascorbinsäure | 0,10 |
| HEDP, wässrig, 60% | 0,20 |
| Natriumsilikat 40/42 | 0,50 |
| Kaliumhydroxid, wässriq, 50% | 1,00 |
| Glycin | 1,00 |
| Taurin | 1,00 |
| alpha-Liponsäure | 0,20 |
| Litchiderm LS 9704 | 1,00 |
| p-Toluylendiaminsulfat | 2,81 |
| 2,4-Diaminophenoxyethanol 2HCl | 0,44 |
| Resorcin | 1,00 |
| m-Aminophenol | 0,20 |
| Monoethanolamin | 9,20 |
| Parfum | qs |
| Wasser, vollentsalzt | Ad 100 |

Rohstoffe: Lanette D (INCI-Bezeichnung: Cetearyl alcohol; Cognis); Lorol C12-18 techn. (INCI-Bezeichnung: Coconut alcohol; Cognis); Eumulgin B 2 (INCI-Bezeichnung: Ceteareth-20; Cognis); Eumulgin B 1 (INCI-Bezeichnung: Ceteareth-12; Cognis); Akypo Soft 45HP (ca. 21 %, INCI-Bezeichnung: Sodium Laureth-6 Carboxylate, Aqua; KAO); Protelan MST 35 (ca. 35 %, INCI-Bezeichnung: Sodium Myristoyl Sarconsinate, Sodium Methyl Cocoyl Taurate, Aqua; Zschimmer & Schwarz); Texapon K 14 S Special (ca. 70 %, INCI-Bezeichnung: Sodium Myreth Sulfate, Aqua; Cognis); Produkt W 37194 (ca. 20 %,INCI-Bezeichnung: Acrylamidopropyltrimonium Chloride / Acrylates Copolymer, Aqua; Stockhausen); Litchiderm LS 9704 (INCI-Bezeichnung: Butylene Glycol; Lichti Chinensis Pericarp Extract; Laboratoires Serobiologiques).

Die Fettbasis wurde zusammen bei 80 °C aufgeschmolzen und mit einem Teil der Wassermenge dispergiert. Anschließend wurden die restlichen Rezepturbestandteile unter Rühren der Reihe nach eingearbeitet. Es wurde mit Wasser auf 100 Gew.-% aufgefüllt und die Formulierung kalt gerührt.

### 2) Entwicklerzubereitungen EW (Tabelle 2; Mengenangaben in Gew.-%)

Folgende Entwicklerzubereitungen wurden mit unterschiedlichen polymeren Verdickungsmitteln hergestellt.

| **Rohstoff** | **E1** | **E2** | **V1** | **V2** |
|---|---|---|---|---|
| Natronlauge, 45 % techn. | 0,73 | 0,73 | 0,73 | 0,73 |
| Dipicolinsäure | 0,10 | 0,10 | 0,10 | 0,10 |
| Dinatriumpyrophosphat | 0,03 | 0,03 | 0,03 | 0,03 |
| HEDP, wässrig, 60 Gew.-% | 1,50 | 1,50 | 1,50 | 1,50 |
| Texapon NSO | 2,00 | 2,00 | 2,00 | 2,00 |
| Dow Corninq DB 110 A | 0,07 | 0,07 | 0,07 | 0,07 |
| Carbomer 954, wässrig, 4 Gew.-% | 36,00 = 1,44 AS* | 45,00 = 1,80 AS* | -- | -- |
| Aculyn 33A | -- | -- | 5,14 = 1,44 AS* | 15,00 = 4,20 AS* |
| Wasserstoffperoxid, wässriq, 50 Gew.-% | 12,00 | 12,00 | 12,00 | 12,00 |
| Wasser, vollentsalzt | Ad 100 | | | |

| | | | | |
|---|---|---|---|---|
| AS* : Aktivsubstanz | | | | |

Rohstoffe: Texapon NSO (ca. 27%, INCI-Bezeichnung: Sodium Laureth Sulfate; Cognis); Dow Corning DB 110 A (INCI-Bezeichnung: Dimethicon; Dow Corning); Carbomer 954 (INCI-Bezeichnung: Carbomer; Lubrizol); Aculyn 33A (ca. 28%; INCI-Bezeichnung: Acrylates Copolymer, Aqua; Rohm & Haas).

### 3) Anwendungsmischungen:

Die Färbecreme FC wurde vor der Anwendung jeweils mit einer der Entwicklerlösungen bei Raumtemperatur im Gewichtsverhältnis von 1:1 versetzt und innig vermischt.

Dabei wurden folgende Mischviskositäten erhalten (Tabelle 3):

| **Färbemittel** | **Anwendungsmischung** | **Verdickungsmittel** | **Mischviskosität* [mPa·s]** |
|---|---|---|---|
| **#1** | FC + E1 (erfindungsqemäß) | 0,72 % AS** Carbomer954 | 12400 |
| **#2** | FC + E2 (erfindunqsgemäß) | 0,90 % AS** Carbomer954 | 21100 |
| **#3** | FC + V1 (nicht erfindungsgemäß) | 0,72 % AS** Aculyn 33 | 2000 |
| **#4** | FC + V2 (nicht erfindungsgemäß) | 2,10 % AS** Aculyn 33 | 17100 |

| | | | |
|---|---|---|---|
| * gemessen mit einem Brookfield-Viskosimeter DV-II+Pro mit Spindel #5 bei 4 Upm bei RT (22°C) in 590 mL Bechergläser, hohe Form. ** Aktivsubstanz | | | |

### 4) Ergebnisse

Aus Tabelle 3 wird deutlich, dass die erfindungsgemäßen Färbezubereitungen #1 und #2 trotz deutlich geringerem Verdickungsmittelanteil gegenüber der Zubereitung #4 eine geeignete Viskosität für die Anwendung auf menschlichen Haaren aufweisen. Wird die Verdickungsmittelmenge in der nicht erfinderischen Färbezubereitung #3 auf die Menge der Zubereitungen #1 und #2 reduziert, wird eine für die Anwendung deutlich zu niedrige Mischviskosität erhalten.

## Patentansprüche

1. Kosmetisches Mittel für die Farbveränderung keratinischer Fasern, welches unmittelbar vor der Anwendung durch Vermischen einer Zubereitung (M1),
enthaltend mindestens einen direktziehenden Farbstoff als farbverändernde Komponente, und einer Oxidationsmittelzubereitung (M2),
enthaltend in einem wässrigen, kosmetischen Träger als chemisches Oxidationsmittel mindestens Wasserstoffperoxid, hergestellt wird
und welches eine Viskosität von 5 bis 100 Pa·s besitzt, gemessen bei 22°C,
**dadurch gekennzeichnet, dass** die Oxidationsmittelzubereitung zusätzlich mindestens ein anionisches, polymeres Verdickungsmittel, ausgewählt aus vernetzten Polyacrylsäuren, die ein Molekulargewicht MW von mindestens 500000 g/mol besitzen, enthält, wobei die Polyacrylsäuren Polymerisate aus Acrylsäure selbst darstellen,
und weiterhin **dadurch gekennzeichnet, dass** die Zubereitung (M1) zusätzlich mindestens ein Alkalisierungsmittel, ausgewählt aus Ammoniak, Natriumhydroxid, Natriumcarbonat, Kaliumhydroxid, Kaliumcarbonat, Natriumhydrogencarbonat, Ammoniumcarbonat, Ammoniumhydrogencarbonat, Arginin, Histidin, Monoethanolamin und/oder 2-Amino-2-methylpropanol, enthält.

2. Mittel nach Anspruch 1, **dadurch gekennzeichnet, dass** die Zubereitung (M2) zusätzlich mindestens ein anionisches Tensid in einem Gewichtsanteil von 0,05 bis 1,5 Gew.-%, bezogen auf das Gesamtgewicht der Zubereitung (M2), enthält.

3. Mittel nach einem der Ansprüche 1 oder 2, **dadurch gekennzeichnet, dass** das Mittel einen pH-Wert von 8,5 bis 11,5 bevorzugt 9,5 bis 11,5 besitzt.

## Claims

1. A cosmetic agent for changing the color of keratin fibers, which is produced immediately before application by mixing a preparation (M1).
containing at least one substantive dye as a color-changing component, and an oxidizing agent preparation (M2),
containing, in an aqueous cosmetic carrier as a chemical oxidizing agent, at least hydrogen peroxide, and which agent has a viscosity of from 5 to 100 Pa·s, measured at 22 °C,
**characterized in that** the oxidizing agent preparation additionally contains at least one anionic, polymer thickening agent selected from crosslinked polyacrylic acids which have a molecular weight MW of at least 500,000 g/mol, the polyacrylic acids themselves constituting polymerizates of acrylic acid,
and further **characterized in that** the preparation (M1) additionally contains at least one alkalizing agent selected from ammonia, sodium hydroxide, sodium carbonate, potassium hydroxide, potassium carbonate, sodium hydrogen carbonate, ammonium carbonate, ammonium hydrogen carbonate, arginine, histidine, monoethanolamine and/or 2-amino-2-methylpropanol.

2. The agent according to claim 1, **characterized in that** the preparation (M2) additionally contains at least one anionic surfactant in a weight proportion of from 0.05 to 1.5 wt.% based on the total weight of the preparation (M2).

3. The agent according to one of claims 1 or 2, **characterized in that** the agent has a pH value of from 8.5 to 11.5, preferably of from 9.5 to 11.5.

## Revendications

1. Agent cosmétique destiné à changer la couleur de fibres de kératine, lequel agent est préparé, immédiatement avant l'utilisation, par mélange d'une préparation (M1), contenant au moins un colorant direct comme composant de changement de couleur, et d'une préparation d'agent d'oxydation (M2), contenant comme agent oxydant chimique dans un support cosmétique aqueux au moins du peroxyde d'hydrogène, et qui a une viscosité de 5 à 100 Pas, mesurée à 22°C, **caractérisé en ce que** la préparation d'agent d'oxydation contient en outre au moins un agent épaississant polymère anionique choisi parmi les acides polyacryliques réticulés qui ont un poids moléculaire MW d'au moins 500000 g/mol les acides polyacryliques sont des polymères de l'acide acrylique lui-même, et **caractérisé en outre en ce que** la préparation (M1) contient en outre au moins un agent d'alcalinisation choisi parmi l'ammoniac, l'hydroxyde de sodium, le carbonate de sodium, l'hydroxyde de potassium, le carbonate de potassium, le bicarbonate de sodium, le carbonate d'ammonium, le bicarbonate d'ammonium, l'arginine, l'histidine, la monoéthanolamine et/ou le 2-amino-2-méthylpropanol.

2. Agent selon la revendication 1, **caractérisé en ce que** la préparation (M2) contient en outre au moins un tensioactif anionique dans une proportion en poids de 0,05 à 1,5% en poids par rapport au poids total de la préparation (M2).

3. Agent selon l'une des revendications 1 ou 2, **caractérisé en ce que** l'agent a une valeur de pH allant de 8,5 à 11,5 de préférence de 9,5 à 11,5.
